# EUROPEAN PATENT SPECIFICATION

(11) **EP 3 642 590 B1**
(45) Date of publication and mention of the grant of the patent: **27.03.2024**
(21) Application number: 18819634.9
(22) Date of filing: 17.06.2018
(51) Int. Cl.: G01N 1/28, G01N 21/13, G01N 21/77, G01N 21/80, G02B 21/34, A61B 10/00, B01L 3/00, G01N 1/30, G01N 1/31, G01N 21/78, G01N 35/00, G01N 1/02

(54) **ASSAY DEVICES AND ASSAY APPARATUS FOR USE THEREWITH**
TESTEINRICHTUNG UND TESTVORRICHTUNG ZUR VERWENDUNG DAMIT
DISPOSITIFS D'ANALYSE ET APPAREIL D'ANALYSE DESTINÉ À ÊTRE UTILISÉ AVEC CEUX-CI

(30) Priority: 21.06.2017 IL 25306717
(43) Date of publication of application: 29.04.2020
(73) Proprietor: GynTools Ltd, 9112001 Jerusalem (IL)
(72) Inventor: LEV-SAGIE, Menachem, 7313300 Lapid (IL); LEV, Nimrod, 5690500 Savion (IL)
(74) Representative: Stütz, Jan
(86) International application number: PCT/IL2018/050671
(87) International publication number: WO 2018/235073

(56) References cited:
- WO-A2-2005/108604
- KR-A- 20140 140 068
- US-A1- 2002 182 739
- US-A1- 2015 094 219
- US-B1- 6 170 318
- US-B1- 8 371 182
- US-B2- 9 518 920
- US-B2- 9 518 920

## Description

### Field of the Invention

This invention relates to assay devices and assay apparatus.

### Background of the Invention

Assays are part of multiple step investigative procedures for qualitatively assessing or quantitatively measuring the presence, amount, or functional activity of one or more specimens. Assays include pre- and post-analytic procedures. Pre- analytic steps include inter alia information processing, specimen handling, and the like. Post-analytic steps include inter alia report documentation, report transmission, and the like. Assays can be employed for investigating chemical specimens, biological specimens, organic specimens and non-organic specimens. Specimens can be solid, liquid, gel, and the like. Investigations can include inter alia Electro-Magnetic Radiation (EMR) examinations at one or more predetermined EMR spectrum including inter alia visual spectrum, IR spectrum, UV spectrum, and the like. Such EMR examinations include inter alia naked eye visual examinations, optical examinations using optical apparatus, for example, microscopes, and the like, and digital scanning for image processing purposes.

WO 2005/108604 A2 discloses a device for the detection of interactions between probe and target molecules, which comprising a microarray comprising a substrate on which probe molecules are immobilized on array elements, said microarray being located on a first surface of the device; and a reaction chamber that is defined between the first area with the microarray disposed thereon and a second surface, the distance between the microarray and the second surface being modifiable. W0 2005/108064 employs the use of a push rod for deflecting a flexible membrane supporting the microarray.

### Summary of the Invention

Generally speaking, the present invention is directed towards assay devices according to claim 1 for use with a handheld specimen handling tool including inter alia a syringe, a pipette, a specimen sampling device, and the like. The specimen handling tool has a leading specimen sample collection end which can take different forms depending on a specimen to be sampled. Suitable specimen sampling collection ends include inter alia a brush, a swab, a shallow bowl, a needle, and the like. The assay devices are designed to be equivalent to a conventional arrangement of a cover slip for placing on a specimen placed on a microscope slide for investigation purposes. The assay devices include a housing having a specimen slide elevation arrangement for elevating a specimen slide from an initial lowermost specimen introduction position to a final uppermost specimen examination position correspondingly remote from and adjacent a housing top face. The housing top face, the specimen slide and a housing bottom face opposite the housing top face each have a panel in mutual registration defining a line of examination therethrough. The panels are transparent to at least one predetermined EMR spectrum. The housing top face panel and the specimen slide panel bound a compression zone therebetween. A handheld specimen handling tool is employed for placing a specimen on the specimen slide panel in its initial lowermost specimen introduction position. The specimen slide elevation arrangement is deployed for elevating the specimen slide to intimately juxtapose its specimen slide panel against the housing top face panel for compressing the specimen therebetween in a similar manner to a conventional arrangement of a microscope slide and a cover slip. The handheld specimen sampling device is necessarily removed from the compression zone during the elevation of the specimen slide.

### Brief Description of Drawings

In order to understand the invention and to see how it can be carried out in practice, preferred embodiments will now be described, by way of non-limiting examples only, with reference to the accompanying drawings in which similar parts are likewise numbered, and in which:
Fig. 1 is a front perspective view of a first preferred embodiment of an assay device with a specimen slide in an initial lowermost specimen introduction position and a handheld specimen handling tool having a specimen;
Fig. 2 is a front perspective view of the Figure 1 assay device with the specimen slide in a final uppermost specimen examination position for naked eye visual examination of the specimen;

The assay devices include a housing having a specimen slide elevation arrangement for elevating a specimen slide from an initial lowermost specimen introduction position to a final uppermost specimen examination position correspondingly remote from and adjacent a housing top face. The housing top face, the specimen slide and a housing bottom face opposite the housing top face each have a panel in mutual registration defining a line of examination therethrough. The panels are transparent to at least one predetermined EMR spectrum. The housing top face panel and the specimen slide panel bound a compression zone therebetween. A handheld specimen handling tool is employed for placing a specimen on the specimen slide panel in its initial lowermost specimen introduction position. The specimen slide elevation arrangement is deployed for elevating the specimen slide to intimately juxtapose its specimen slide panel against the housing top face panel for compressing the specimen therebetween in a similar manner to a conventional arrangement of a microscope slide and a cover slip. The handheld specimen sampling device is necessarily removed from the compression zone during the elevation of the specimen slide.

### Brief Description of Drawings

In order to understand the invention and to see how it can be carried out in practice, preferred embodiments will now be described, by way of non-limiting examples only, with reference to the accompanying drawings in which similar parts are likewise numbered, and in which:

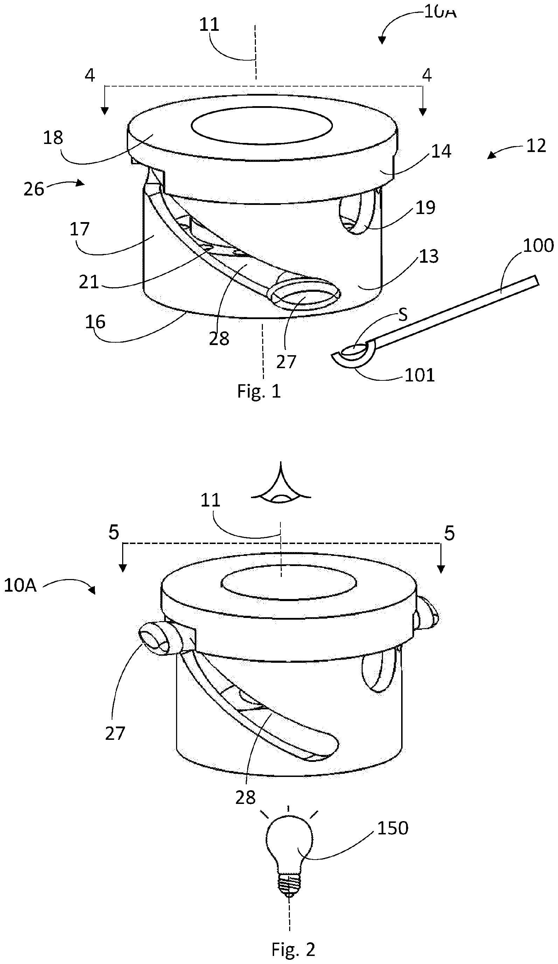

Fig. 1 is a front perspective view of a first preferred embodiment of an assay device with a specimen slide in an initial lowermost specimen introduction position and a handheld specimen handling tool having a specimen;
Fig. 2 is a front perspective view of the Figure 1 assay device with the specimen slide in a final uppermost specimen examination position for naked eye visual examination of the specimen;

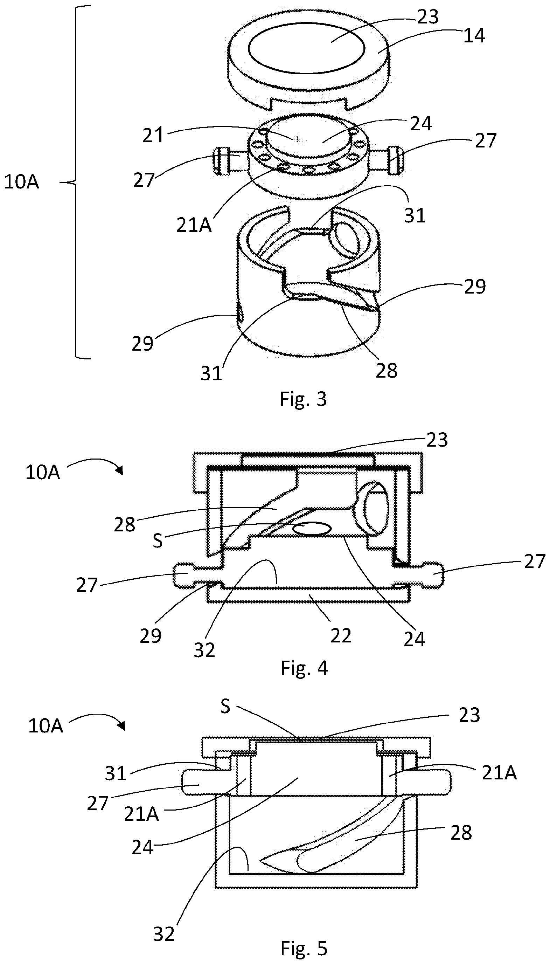

Fig. 3 is an exploded view of the Figure 1 assay device;
Fig. 4 is a longitudinal cross section of the Figure 1 assay device after placing the specimen on the specimen slide along line 4-4 in Figure 1;
Fig. 5 is a longitudinal cross section of the Figure 1 assay device along line 5-5 in Figure 2;

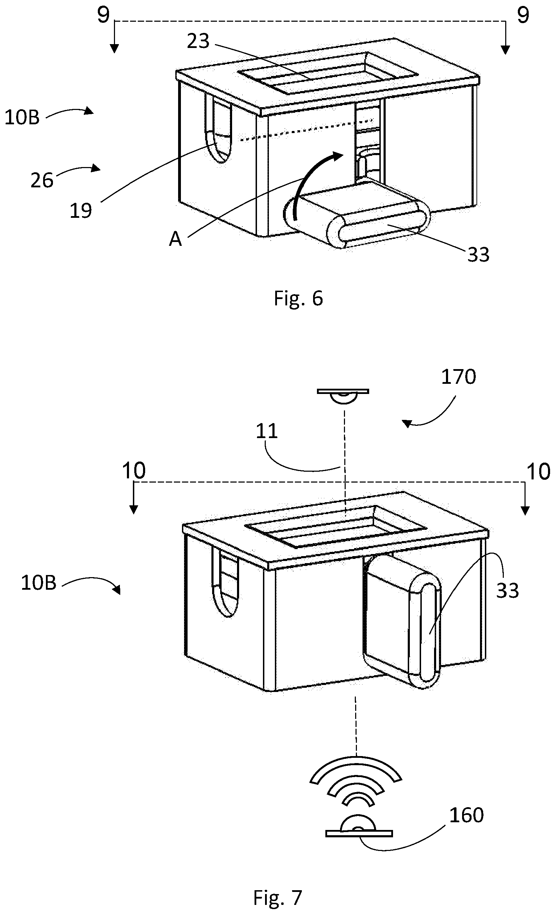

Fig. 6 is a front perspective view of a second preferred embodiment of an assay device with a specimen slide in an initial lowermost specimen introduction position;
Fig. 7 is a front perspective view of the Figure 6 assay device with the specimen slide in a final uppermost specimen examination position for IR imaging of the specimen;

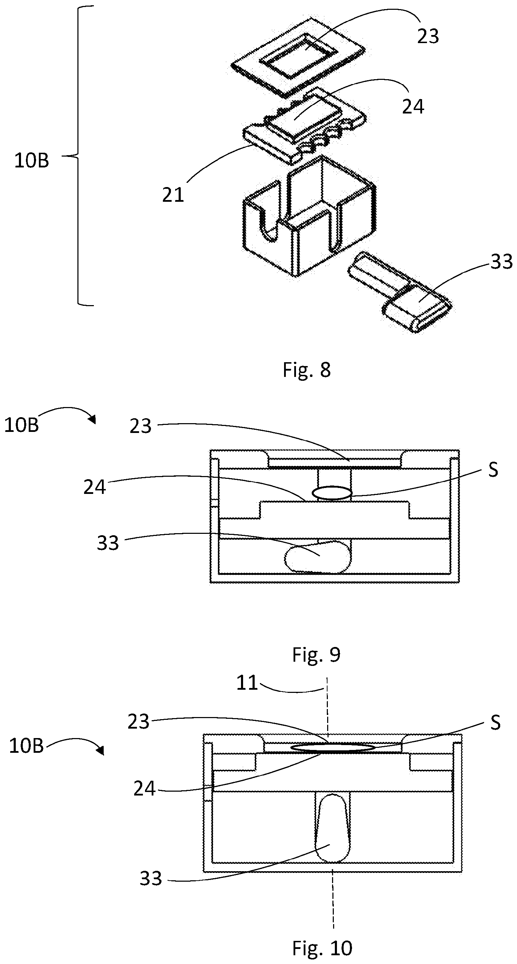

Fig. 8 is an exploded view of the Figure 6 assay device;
Fig. 9 is a longitudinal cross section of the Figure 6 assay device along line 9-9 in Figure 6;
Fig. 10 is a longitudinal cross section of the Figure 6 assay device along line 10-10 in Figure 7;

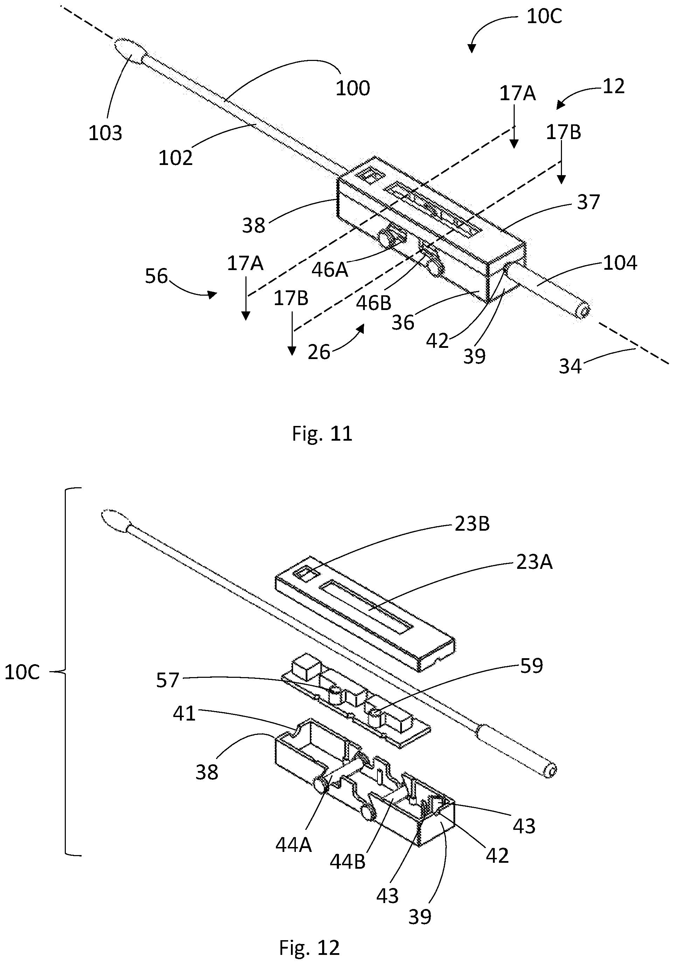

Fig. 11 is a front perspective view of a third preferred embodiment of an assay device with a specimen slide in an initial lowermost specimen introduction position and a pre-deployed handheld pull through elongated swab having a specimen;
Fig. 12 is an exploded view of the Figure 11 assay device;

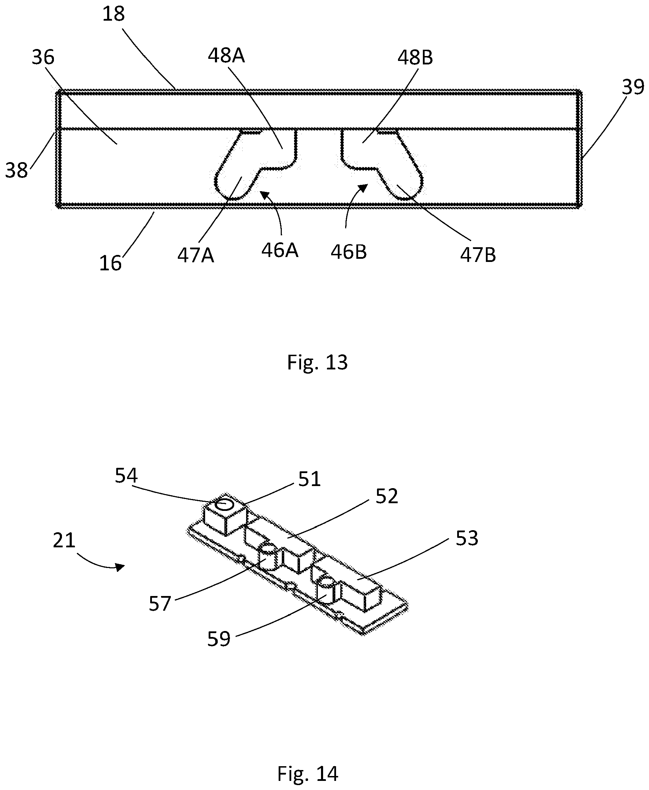

Fig. 13 is a front elevation view of the Figure 11 assay device's housing;
Fig. 14 is a front perspective view of the Figure 11 assay device's specimen slide;

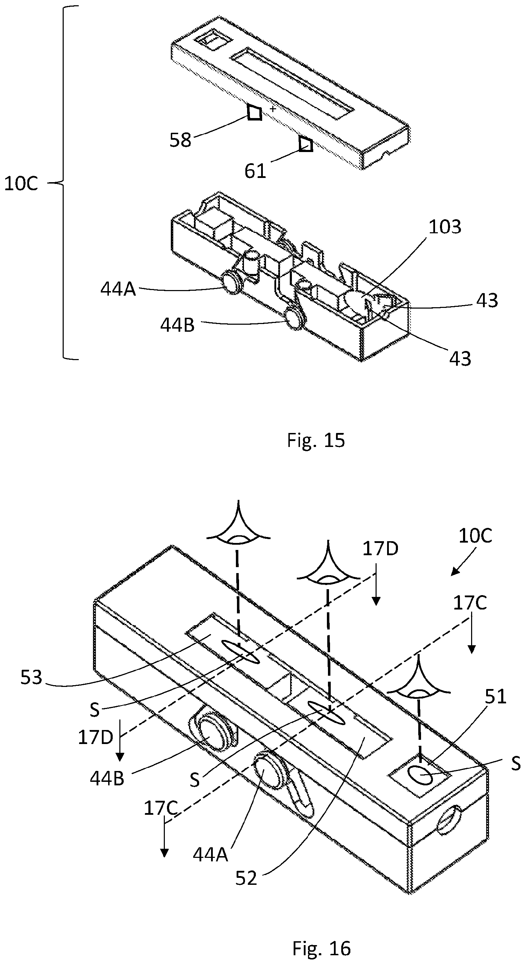

Fig. 15 is a partially exploded view of the Figure 11 assay device with the specimen slide in the initial lowermost specimen examination position;
Fig. 16 is a front perspective view of the Figure 11 assay device with the specimen slide in a final uppermost specimen examination position for naked eye visual examination;

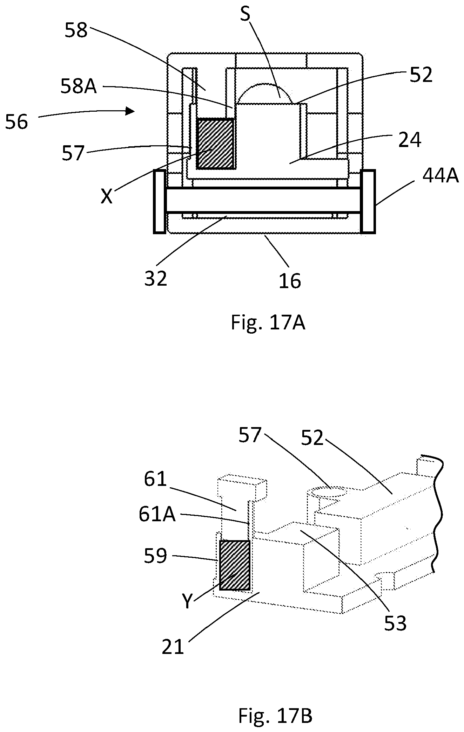

Fig. 17A is a transverse cross section of the Figure 11 assay device along line 17A-17A in Figure 11;
Fig. 17B is a partial cut-away view of the Figure 11 assay device with the specimen slide in its initial lowermost specimen introduction position along line 17B-17B in Figure 11;

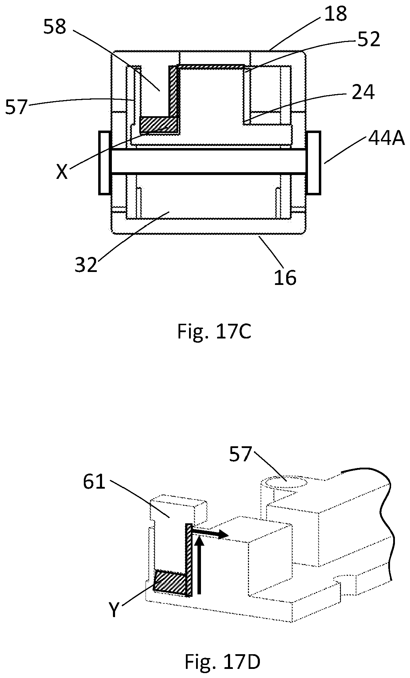

Fig. 17C is a transverse cross section of the Figure 11 assay device along line 17C-17C in Figure 16;
Fig. 17D is a partial cut-away view of the Figure 11 assay device with the specimen slide in its final uppermost specimen examination position along line 17D-17D in Figure 16;

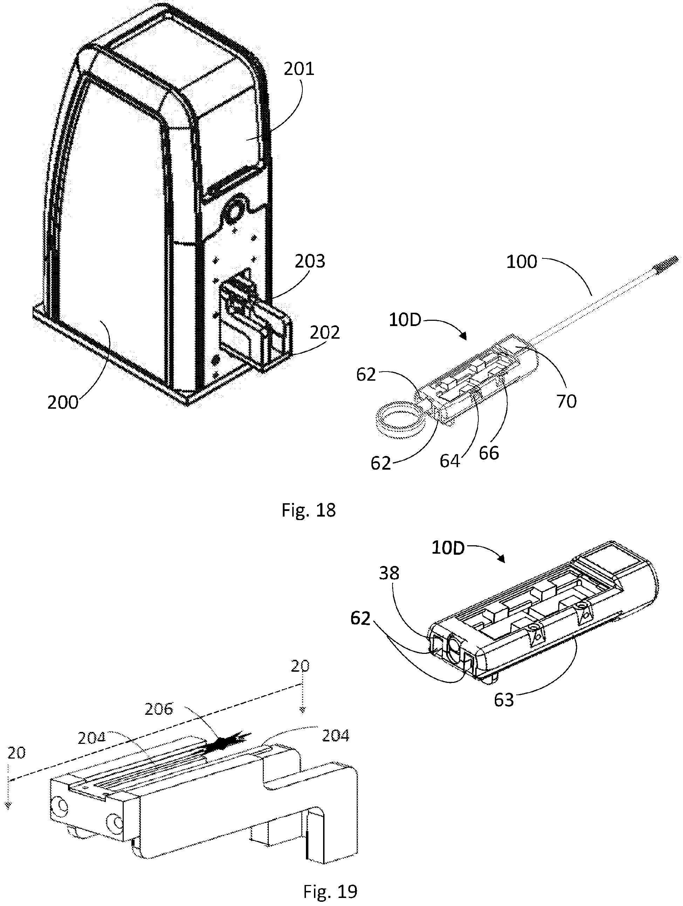

Fig. 18 is a front perspective view of a fourth preferred embodiment of an assay device for use with assay apparatus;
Fig. 19 is a close-up view of a specimen slide elevation member of the Figure 18 assay apparatus;

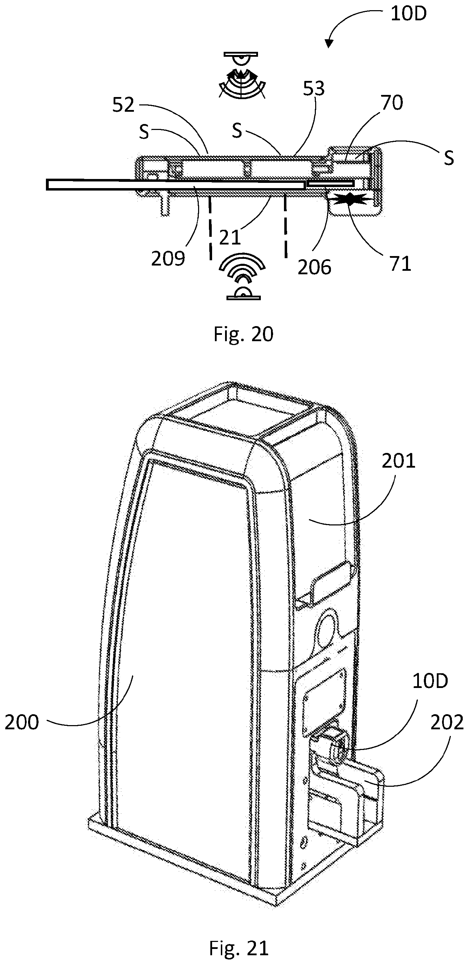

Fig. 20 is a longitudinal cross section of the Figure 18 assay device mounted on the specimen slide elevation member along line 20-20 in Figure 19; and
Fig. 21 is a perspective view of the assay apparatus with the Figure 18 assay device inserted for specimen examination.

### Detailed Description of Drawings

Figure 1 to Figure 5 show an assay device 10A for use with a handheld specimen handling tool 100 in the form of a spoon with a leading specimen collection end 101 in the form of a shallow bowl. The bowl 101 contains a specimen S. The assay device 10A affords a line of examination 11 therethrough for examination of a specimen inserted thereinto.

The assay device 10A includes a dual component cylindrical housing 12 having a base 13 and a cover 14. The base 13 constitutes a housing bottom face 16 and a housing peripheral face 17. The cover 14 constitutes a housing top face 18 opposite the housing bottom face 16. The housing peripheral face 17 is formed with a specimen introduction port 19 for enabling introduction of a specimen into the housing 12.

The assay device 10A includes a specimen slide 21 below the specimen introduction port 19 in an initial lowermost specimen introduction position (see Figure 1 and Figure 4). The housing bottom face 16 has a transparent housing bottom face panel 22, the housing top face 18 has a transparent housing top face panel 23 and the specimen slide 21 has a transparent specimen slide panel 24 in mutual registration for enabling the line of examination 11. The housing top face panel 23 and the specimen slide panel 24 define a compression zone therebetween. The panels 22, 23 and 24 are transparent to the visible spectrum for naked eye examination through the assay device 10A along the line of examination 11. The specimen can be additionally backlighted from a visible light source 150 under the assay device 10A.

The assay device 10A includes a specimen slide elevation arrangement 26 for elevating the specimen slide 21 from its initial lowermost specimen introduction position (see Figure 1 and Figure 4) to a final uppermost specimen examination position (see Figure 2 and Figure 5) correspondingly remote from and adjacent the housing top face 18. The specimen slide elevation arrangement 26 is constituted by a manually operated specimen slide elevation mechanism in the form of the specimen slide 21 having a diametric pair of outward radial specimen slide elevation members 27 extending through a diametric pair of elevation tracks 28 formed in the housing peripheral face 17. The diametric pair of elevation tracks 28 each spiral from a lowermost elevation track end 29 adjacent the housing bottom face 16 to an uppermost elevation track end 31 adjacent the housing top face 18. The elevation tracks 28 are preferably about a quarter turn in length.

Deployment of the diametric pair of specimen slide elevation members 27 at the lowermost elevation track ends 29 corresponds to the initial lowermost specimen introduction position. Deployment of the diametric pair of specimen slide elevation members 27 at the uppermost elevation track ends 31 corresponds to the final uppermost specimen examination position. The specimen slide elevation arrangement 26 enables a user to impart a rotation movement of the specimen slide 21 relative to the housing 12 for elevating the specimen slide 21 from its initial lowermost specimen introduction position to its final uppermost specimen examination position.

In the final uppermost specimen examination position, the transparent specimen slide panel 24 is intimately juxtaposed against the transparent housing top face panel 23 for compressing the specimen S therebetween. Compression of a specimen thinly spreads the specimen similar to a conventional arrangement of a cover slip on a microscope slide. Assay devices 10A can be designed for applying different degrees of compression of different specimens. The housing bottom face 16 can be lined with hygroscopic material 32 to absorb liquid residues displaced from the specimen slide 21 pursuant to its elevation to its final uppermost specimen examination position. The liquid residues can include specimen residues and reagent residues in the case of dispensing a liquid reagent on a specimen for reacting with same. The specimen slide 21 is preferably provided with an annular arrangement of drainage holes 21A for facilitating drainage from the specimen slide panel 24 to the hygroscopic material 32.

The use of the assay device 10A is as follows: A user uses the handheld specimen handling tool 100 to collect a specimen S. The user introduces the specimen S into the housing 12 and deposits at least some of the specimen S on the specimen slide panel 24. The user withdraws the specimen handling tool 100 from the compression zone in order not to obstruct elevation of the specimen slide 21. The user can optionally introduce a liquid reagent dispenser through the specimen introduction port 19 for dispensing liquid reagent on the specimen for reacting with same or diluting same.

The user grips the housing 12 in one hand and uses his other hand to impart a rotation movement of the specimen slide 21 relative to the housing 12 for elevating the specimen slide 21 from its initial lowermost specimen introduction position to its final uppermost specimen examination position for compressing the specimen S against the housing top face panel 23. The user can view the specimen S through the housing top face panel 23 for visual examination of same. The user can place the assay device 10A on a visible light illumination source 150 for backlighting the specimen S for assisting visual examination. Alternatively, the user can place the assay device 10A on a microscope in a similar manner as a conventional microscope slide for optical examination of the specimen S.

Figure 6 to Figure 10 show an assay device 10B similar in construction to the assay device 10A and therefore similar parts are likewise numbered. The assay device 10B differs from the assay device 10A in three respects as follows: First, the specimen slide elevation arrangement 26. Second, the assay device 10B is intended for IR examination of a specimen and not naked eye visual examination. Accordingly, the assay device 10B can be opaque to the naked eye. And third, the assay device 10B can have different shapes including *inter alia* square, rectangle, ellipsoidal, and the like.

The assay device 10B has a specimen slide elevation arrangement 26 constituted by a specimen slide elevation actuator 33 deployed under the specimen slide 21 in its initial lowermost specimen introduction position. The specimen slide elevation actuator 33 has an initial set-up position and a final examination position respectively corresponding to the specimen slide's initial lowermost specimen introduction position (see Figure 6 and Figure 9) and a final examination position corresponding to specimen slide's final uppermost specimen examination position (see Figure 7 and Figure 10). A user rotates the specimen slide elevation actuator 33 through a quarter clockwise turn A from its initial set-up position to its final examination position.

The use of the assay device 10B is similar to the assay device 10A except that a user rotates the specimen slide elevation actuator 33 rather than rotating the specimen slide 21 relative to the housing 12. The user places the assay device 10B between an IR radiation source 160 and an IR radiation detector 170. The IR radiation detector 170 detects IR radiation from the IR radiation source 160 which passes through the assay device 10B and its specimen along the line of examination 11.

Figure 11 to Figure 17 show an assay device 10C similar in construction to the assay device 10A and therefore similar parts are likewise numbered. The assay device 10C is supplied with a pre-deployed handheld specimen handling tool 100 in the form of an elongated pull through swab. The pull through swab 100 includes a shank 102, a leading swab head 103 and a trailing handle 104. The swab head 103 has a greater diameter than the shank 102 and is preferably intentionally detachable therefrom on application of a relatively small detachment force. Such detachment promotes safe handling of the assay device 10C by precluding contact with the swab head 103.

The assay device 10C includes a box like housing 12 having a longitudinal assay device centerline 34, a major front face 36, a major back face 37 opposite the major front face 36, a leading minor end face 38 and a trailing minor end face 39 opposite the leading minor end face 38. The leading minor end face 38 and the trailing minor end face 39 are correspondingly formed with a leading minor end face port 41 and a trailing minor end face port 42. The leading minor end face port 41 has a greater diameter than the swab head 103 such that the swab head 103 can be pulled into the assay device 10C. The trailing minor end face port 42 is formed with a pair of abutment members 43 defining an opening therebetween smaller than the swab head 103 such that the swab head 103 detaches from the shank 102 on abutment thereagainst on pulling the swab 100 through the assay device 10C. The swab 100 has an initial set-up position in the assay device 10C in which its swab head 103 is remote from the leading minor end face 38 and its handle 104 is adjacent the trailing minor end face 39. The swab head 103 can typically protrude by say 15 cm from the leading minor end face 38 in the initial set-up position.

The assay device 10C includes a specimen slide elevation arrangement 26 constituted by a leading specimen slide elevation actuator 44A adjacent the leading minor end face 38 and a trailing specimen slide elevation actuator 44B adjacent the trailing minor end face 39. The leading specimen slide elevation actuator 44A extends widthwise across the housing 12 and protrudes from an opposite pair of specimen slide elevation tracks 46A in the major front face 36 and the major back face 37 for manual actuation purposes. The specimen slide elevation tracks 46A each have a lower inclined elevation leg 47A and an upper horizontal locking leg 48A adjacent the housing top face 18 and extending from its lower inclined elevation leg 47A towards the trailing minor end face 39. The trailing specimen slide elevation actuator 44B extends widthwise across the housing 12 and protrudes from an opposite pair of specimen slide elevation tracks 46B in the major front face 36 and the major back face 37 for manual actuation purposes. The specimen slide elevation tracks 46B each has an inclined elevation leg 47B and a horizontal locking leg 48B adjacent the housing top face 18 and extending from its lower inclined elevation leg 47B towards the leading minor end face 38. The inclined elevation legs 47A and the inclined elevation legs 47B converge towards one another from the housing bottom face 16 towards the housing top face 18 such that the specimen slide elevation actuators 44A and 44B have an initial set-up position adjacent the housing bottom face 16 and a final examination position towards the housing top face 18. The initial set-up position corresponds to the initial lowermost specimen introduction position and the final examination position corresponds to the final uppermost specimen examination position.

The assay device 10C includes a specimen slide 21 having three discrete work surfaces as follows: a leading work surface 51 adjacent the leading minor end face 38, a central work surface 52 midway between the leading minor end face 38 and the trailing minor end face 39 and a trailing work surface 53 adjacent the trailing minor end face 39. The assay device 10C is designed such that the swab end 103 smears specimen on the work surfaces 51, 52 and 53 as the swab 100 is pulled from its initial set-up position through the assay device 10C to withdraw the shank 102 therefrom leaving the swab head 103 in the housing 12. The leading work surface 51 has a pH detection surface 54 for providing a visible color indication regarding the pH value of a specimen smeared thereon. The leading work surface 51 is not necessarily transparent as opposed to the central work surface 52 and the trailing work surface 53 are necessarily transparent to afford parallel lines of examination through the assay device 10C. The transparent housing top face panel 23 can extend lengthwise along the three work surfaces 51, 52, and 53 or alternatively be formed with individual sub-panels 23A and 23B each covering a single work surface.

The assay device 10C includes an integral liquid reagent pump arrangement 56 for pumping one or more liquid reagents for reacting with a corresponding number of specimen samples during elevation of the specimen slide 21 to its final uppermost specimen examination position. The liquid reagents may change color on reaction with the specimen samples or undergo another discernible change. The liquid reagent pump arrangement 56 includes a central liquid reagent reservoir 57 containing a liquid reagent X and a central plunger 58 for inserting in the central liquid reagent reservoir 57 for pumping liquid reagent X therefrom onto the specimen on the central work surface 52. The central liquid reagent reservoir 57 typically contains, for example, saline. The liquid reagent pump arrangement 56 includes a trailing liquid reagent reservoir 59 containing a liquid reagent Y and a trailing plunger 61 for inserting in the trailing liquid reagent reservoir 59 for pumping liquid reagent Y therefrom onto the specimen on the trailing work surface 53. The trailing liquid reagent reservoir 59 contains, for example, potassium hydroxide. The central liquid reagent reservoir 57 and the trailing liquid reagent reservoir 59 are formed on the specimen slide 21. The central plunger 58 and the trailing plunger 61 are downward depending from the housing top face 18 in the direction of the housing bottom face 16.

The central plunger 58 is provided with a central plunger groove 58A for pumping liquid reagent X from the central liquid reagent reservoir 57 onto the central work surface 52 as the specimen slide 21 is displaced from its initial lowermost specimen introduction position to its final uppermost specimen examination position. The trailing plunger 61 is similarly provided with a trailing plunger groove 61A for pumping liquid reagent Y from the trailing liquid reagent reservoir 59 onto the trailing work surface 53 as the specimen slide 21 is displaced from its initial lowermost specimen introduction position to its final uppermost specimen examination position.

Figure 17A shows the central liquid reagent reservoir 57 filled with liquid reagent X and the central plunger 58 in its set-up position. Figure 17B shows the trailing liquid reagent reservoir 59 filled with liquid reagent Y and the trailing plunger 61 in its set-up position. Figure 17C shows elevation of the specimen slide 21 from its initial lowermost specimen introduction position to its final uppermost specimen examination position urges the central plunger 58 into the central liquid reagent reservoir 57 for dispensing liquid reagent X via the central plunger groove 58A on the specimen on the central work surface 52. Similarly, Figure 17D shows the trailing plunger 61 being urged into the trailing liquid reagent reservoir 59 for dispensing liquid reagent Y via the trailing plunger groove 61A on the specimen on the trailing work surface 53.

The use of the assay device 10C is similar to the assay device 10A. A user collects specimen on the swab head 103 and pulls the swab 100 through the assay device 10C such that the swab head 103 passes through the leading minor end face port 41 before smearing specimen on the leading work surface 51, the central work surface 52 and the trailing work surface 53. The user continues to pull on the handle 104 such that the swab head 103 abuts against the abutment members 43 and detaches from the shank 102.

The pH detection surface 54 changes color in accordance with the specimen's pH and is visible through the housing top face panel 23. The user holds the housing 12 in one hand and uses his other hand's forefinger and thumb to displace the specimen slide elevation actuator 44A and the specimen slide elevation actuator 44B towards one another. Such displacement initially correspondingly pumps liquid reagents from the central liquid reagent reservoir 57 and the trailing liquid reagent reservoir 59 onto the central work surface 52 and trailing work surface 53 for reaction with specimen smeared thereon. Such displacement ends with the specimen slide panel 24 juxtaposed against the housing top face panel 23. Excess liquid reagent and specimen are drained from the central work surface 52 and the trailing work surface 53 onto the hygroscopic material 32 lining the housing bottom face 16. The user can visually examine the reacted specimens on the central work surface 52 and the trailing work surface 53 along their corresponding lines of examination 11 through the assay device 10C. Alternatively, the reacted specimens can be examined under non-visible EMR spectrum, and the like.

Figure 18 to Figure 21 show an assay device 10D similar in construction to the assay device 10C and therefore similar parts are likewise numbered. The assay device 10D is also supplied with a pre-deployed handheld specimen handling tool 100 in the form of an elongated pull through swab. The assay device 10D is intended to be used with assay apparatus 200 in the form of a digital microscope, and the like. Suitable digital microscopes include *inter alia* Leica DVM6, and the like. The assay apparatus 200 can be a standalone device with built-in diagnosis capabilities for examining a specimen. Alternatively, the assay apparatus 200 can transmit images and other information acquired from a specimen for remote processing.

The assay apparatus 200 includes an assay apparatus housing 201 and a reciprocal assay device tray 202. The assay apparatus 200 is preferably operative similar to a personal computer having a disc drive insofar as the assay device tray 202 has an assay device access position (see Figure 18) and an assay device examination position (see Figure 21). The assay device tray 202 protrudes further from the assay apparatus housing 201 in the assay device access position than the assay device examination position. The assay device tray 202 enables placement of an assay device 10D thereon in the assay device access position and removal therefrom. The assay device tray 202 is inserted in the assay apparatus housing 201 for specimen examination purposes. The assay device tray 202 includes a specimen slide elevation member 203 for insertion into an assay device 10D for elevating its specimen slide 21 from its initial lowermost specimen introduction position to its final uppermost specimen examination position. The specimen slide elevation member 203 is preferably forked shape with spaced apart specimen slide elevation prongs 204 which do not obstruct lines of examination 11 through the assay device 10D. The specimen slide elevation member 203 preferably includes a multi-pin connector 206 for connection to a matching assay device multi-pin connector.

The assay device 10D differs from the assay device 10C into three respects as follows: First, the assay device 10D does not include a manual operated specimen slide elevation arrangement. As an alternative for use with the assay apparatus 200 with the specimen slide elevation member 203, a discrete specimen slide elevation member 203 can be inserted in the assay device 10D for elevating its specimen slide 21 from its initial lowermost specimen introduction position to its final uppermost specimen examination position. Second, the assay device 10D requires manual introduction of liquid reagents instead of the liquid reagent pump arrangement. And third, the assay device 10D includes a commercially available so-called electrochemical nose for obtaining odor information from a specimen. Such electrochemical noses are also known as e-nose and micro nose. Commercially available electrochemical noses include *inter alia* Figaro TGS8100, and the like.

The leading minor end face 38 is formed with a slot pair 62 spaced apart across its width and under the specimen slide 21 in its initial lowermost specimen introduction position. The slot pair 62 is intended to slidingly receive the specimen slide elevation member 203 therein. The housing 12 is formed with a specimen slide guidance track 63 for guiding the specimen slide 21 from its initial lowermost specimen introduction position to its final uppermost specimen examination position on the sliding insertion of the specimen slide elevation member 203 thereinto.

The assay device 10D includes a housing top face 18 with two liquid reagent ports 64 and 66 for introducing two liquid reagents for reacting with specimens on the central work surface 52 and the trailing work surface 53.

The assay device 10D includes an electrochemical nose 70 for obtaining odor information from the specimen S and a matching multi-pin connector 71 to the multi-pin connector 206. The multi-pin connectors 206 and 71 can include power lines for powering the electrochemical nose 70 and data lines for odor information regarding the specimen S.

The use of the assay device 10D is the same as the assay device 10C except that a user is required to manually add liquid reagent to the specimens on the central work surface 52 and trailing work surface 53. Also the user is required to mount the assay device 10D onto the assay device tray 202 ensuring correct insertion of the specimen slide elevation member 203 thereinto as opposed to actuating the specimen slide elevation actuators 44. On operation of the assay apparatus 200 to draw the assay tray 202 into the assay apparatus housing 201, the assay apparatus 200 examines the reacted specimens on the central work surface 52 and the trailing work surface 53 along their corresponding lines of examination 11 through the assay device 10D. Also, the assay apparatus 200 detects the specimen's pH value from the pH detection surface 54.

While the invention has been described with respect to a limited number of embodiments, it will be appreciated that many variations, modifications, and other applications of the invention can be made within the scope of the appended claims.

## Claims

1. An assay device (10A, 10B, 10C, 10D) for enabling Electro-Magnetic Radiation, EMR, examination of a specimen at at least one predetermined EMR spectrum, the assay device comprising:
a) a handheld housing (12) having a housing bottom face (16), a housing top face (18) opposite said housing bottom face (16) , and a housing peripheral face (17),
said housing (12) having a specimen introduction port (19) in said housing peripheral face (17) for enabling introduction of the specimen thereinto,
said housing bottom face (16) having a housing bottom face panel (22) and said housing top face (18) having a housing top face panel (23) in registration with said housing bottom face panel (22) for defining a line of examination through the assay device,
said housing bottom face panel (22) and said housing top face panel (23) being transparent to the at least one predetermined EMR spectrum;
b) a specimen slide (21) having a specimen slide panel (24) for at least some specimen to be deposited thereon,
said specimen slide panel (24) being transparent to the at least one predetermined EMR spectrum and in mutual registration with said housing top face panel (23) and said housing bottom face panel (22) along said line of examination; and
c) a specimen slide elevation arrangement for elevating said specimen slide, whereby said specimen slide (21) is adapted to be slidingly elevated with respect to said housing bottom face from an initial lowermost specimen introduction position to a final uppermost specimen examination position correspondingly remote from and adjacent said housing top face (18) subsequent to said at least some specimen being deposited on said specimen slide panel (24) in said initial lowermost specimen introduction position, and whereby said specimen slide panel (24) is adapted to be intimately juxtaposed against said housing top face panel (23) in said final uppermost specimen examination position for compressing said at least some specimen between said specimen slide panel (24) and said housing top face panel (23).

2. The assay device (10A, 10B, 10C, 10D) according to claim 1 wherein said specimen slide (21) includes a pH chemical detector (54) for providing a visible color indication regarding the specimen's pH value.

3. The assay device (10A, 10B, 10C, 10D) according to either claim 1 or 2 wherein said housing bottom face (16) is lined with hygroscopic material to absorb liquid residues displaced from said specimen slide (21) pursuant to elevation of said specimen slide (21) to said final uppermost specimen examination position.

4. The assay device (10A, 10B, 10C, 10D) according to any one of claims 1 to 3 wherein said housing top face (18) includes a liquid reagent port (64, 66) and a liquid reagent channel for guiding liquid reagent onto said specimen slide (21) in said initial lowermost specimen introduction position to react with the at least some specimen on said specimen slide panel (24).

5. The assay device (10A, 10B, 10C, 10D) according to any one of claims 1 to 3 wherein said housing (12) includes an integral liquid reagent pump arrangement (56) for pumping liquid reagent on said specimen slide to react with the at least some specimen in said initial lowermost specimen introduction position, said integral liquid reagent pump arrangement including a reagent reservoir containing liquid reagent and said housing top face include a downward depending plunger whereupon elevation of said specimen slide urges said plunger into said reagent reservoir for pumping liquid reagent from said reagent reservoir onto said specimen slide panel.

6. The assay device (10A, 10B, 10C, 10D) according to any one of claims 1 to 5 wherein said housing (12) includes at least one manually operated specimen slide elevation actuator (44) under said specimen slide (21) in said initial lowermost specimen introduction position, said at least one manually operated specimen slide elevation actuator (44) having an initial set-up position corresponding to said initial lowermost specimen introduction position and a final examination position corresponding to said final uppermost specimen examination position.

7. The assay device (10A, 10B, 10C, 10D) according to claim 6 wherein said housing (12) includes
a leading manual operated specimen slide elevation actuator (44) under said specimen slide in said initial lowermost specimen introduction position for sliding along a leading opposite pair of specimen slide elevation tracks (28) in said housing peripheral face (17) each including at least an inclined elevation leg, and
a trailing manual operated specimen slide elevation actuator under said specimen slide in said initial lowermost specimen introduction position for sliding along a trailing opposite pair of specimen slide elevation tracks in said housing peripheral face each including at least an inclined elevation leg (47), said leading manual operated specimen slide elevation actuator (44) and trailing manual operated specimen slide elevation actuator being spaced apart for manual displacement along their corresponding opposite pairs of specimen slide elevation tracks (28) towards one another from an initial set-up position to a final examination position, said initial set-up position corresponding to said initial lowermost specimen introduction position and said final examination position corresponding to said final uppermost specimen examination position.

8. The assay device (10A, 10B, 10C, 10D) according to any one of claims 1 to 5 wherein said housing (12) includes i) a slot (62) in said housing peripheral face (17) under said specimen slide (21) in said initial lowermost specimen introduction position for sliding insertion of a specimen slide elevation member (203) into said housing (12) and ii) a specimen slide guidance track (63) for guiding said specimen slide (21) from said initial lowermost specimen introduction position to said final uppermost specimen examination position on said sliding insertion of said specimen slide elevation member (203) into said housing (12).

9. Assay apparatus (200) for use with the assay device (10A, 10B, 10C, 10C) according to claim 8 for obtaining an image of the specimen wherein the assay apparatus (200) includes said specimen slide elevation member (203) for inserting in the assay device (10A, 10B, 10C, 10D).

10. Assay apparatus (200) according to claim 9 wherein the assay device (10A, 10B, 10C, 10D) includes an odor sensor (70) for obtaining odor information from the specimen.

## Patentansprüche

1. Untersuchungsvorrichtung (10A, 10B, 10C, 10D) zur Untersuchung einer Probe mit elektromagnetischer Strahlung, EMR, in mindestens einem vorbestimmten EMR-Spektrum; die Untersuchungsvorrichtung umfassend:
a) ein handhaltbares Gehäuse (12) mit einer Gehäuseunterseite (16) und einer Gehäuseoberseite (18) gegenüberliegend der Gehäuseunterseite (16), sowie einer Gehäuseumfangsfläche (17), wobei das Gehäuse (12) eine Probeneinführungsöffnung (19) in der Gehäuseumfangsfläche (17) aufweist, um darin eine Probe einführen zu können, die Gehäuseunterseite (16) mit einer Gehäuseunterseitenplatte (22) und die Gehäuseoberseite (18) mit einer Gehäuseoberseitenplatte (23) in Deckung mit der Gehäuseunterseitenplatte (22) versehen sind, um eine Untersuchungslinie durch die Testvorrichtung zu definieren; wobei die Gehäuseunterseitenplatte (22) und die Gehäuseoberseitenplatte (23) für mindestens ein vorbestimmtes EMR-Spektrum durchlässig sind;
b) einen Objektträger (21) mit einer Probenplatte (24), zur Aufnahme einer Probe, wobei die Probenplatte (24), wenigstens für das vorbestimmte EMR-Spektrum durchlässig ist und sich in gegenseitiger Deckung mit der Gehäuseoberseitenplatte (23) und der Gehäuseunterseitenplatte (22) entlang der Untersuchungslinie befinden; und
c) eine Anordnung zum Anheben des besagten Objektträgers wobei der Objektträger (21) so geformt ist, dass dieser gegenüber der Gehäuseunterseite gleitend von einer anfänglichen untersten Probeneinführungsposition zu einer endgültigen obersten Probenuntersuchungsposition geschoben werden kann, entsprechend beabstandet von und anliegend an die Gehäuseoberseite (18) nachdem wenigstens eine Probe auf der Probenplatte (24) in der anfänglichen Probeneinführungsposition positioniert wurde und wobei die Probenplatte (24) dazu ausgebildet ist, dass sie in der endgültigen obersten Probenuntersuchungsposition eng an die Gehäuseoberseitenplatte (23) angrenzt, um die wenigstens eine Probe zwischen der Probenplatte (24) und der Gehäuseoberseitenplatte (23) zu komprimieren.

2. Untersuchungsvorrichtung (10A, 10B, 10C, 10D) nach Anspruch 1, wobei der Objektträger (21) einen chemischen pH-Detektor (54) enthält, der eine sichtbare Farbanzeige für den pH-Wert der Probe bereitstellt.

3. Untersuchungsvorrichtung (10A, 10B, 10C, 10D) nach einem der Ansprüche 1 oder 2, wobei die Gehäuseunterseite (16) mit einem hygroskopischen Material bekleidet ist, um Flüssigkeitsausscheidungen zu absorbieren, die von dem Objektträger (21) infolge seines Anhebens in die endgültige, oberste Probenuntersuchungsposition verdrängt werden.

4. Untersuchungsvorrichtung (10A, 10B, 10C, 10D) nach einem der Ansprüche 1 bis 3, wobei die Gehäuseoberseite (18) eine Öffnung für ein flüssiges Reagenz (64, 66) und einen Kanal für ein flüssiges Reagenz zum Leiten von flüssigem Reagenz auf den Objektträger (21) in seiner anfänglichen untersten Probeneinführungsposition besitzt, um anschließend mit wenigstens einer Probe auf der Probenplatte (24) zu reagieren.

5. Untersuchungsvorrichtung (10A, 10B, 10C, 10D) nach einem der Ansprüche 1 bis 3, wobei das Gehäuse (12) eine integrierte Pumpenanordnung für ein flüssiges Reagenz (56) zum Pumpen eines flüssigen Reagenz auf den Objektträger enthält, um mit der wenigstens einen Probe bereits in der anfänglichen untersten Probeneinführungsposition zu reagieren, wobei die integrierte Pumpenanordnung für flüssiges Reagenz ein Reservoir mit flüssigem Reagenz besitzt, und die Gehäuseoberseite einen nach unten hängenden Kolben enthält, in der Form dass das Anheben des Objektträgers den Kolben in das Reservoir mit flüssigem Reagenz drückt, um flüssiges Reagenz aus besagtem Reservoir auf die Probenplatte zu pumpen.

6. Untersuchungsvorrichtung (10A, 10B, 10C, 10D) nach einem der Ansprüche 1 bis 5, wobei das Gehäuse (12) mindestens einen manuell betätigten Objektträger-Anhebe-Aktuator (44) unterhalb des Objektträgers (21) in seiner anfänglichen untersten Probeneinführungsposition besitzt, wobei besagter manueller Obj ektträger-Anhebe-Aktuator (44) eine anfängliche Startposition bereitstellt, die der anfänglichen untersten Probeneinführungsposition entspricht, und eine endgültige Untersuchungsposition, die der finalen obersten Probenuntersuchungsposition entspricht.

7. Untersuchungsvorrichtung (10A, 10B, 10C, 10D) nach Anspruch 6, wobei das Gehäuse (12) einen führenden manuellen Objektträger-Anhebe-Aktuator (44) unter dem Objektträger in der anfänglichen untersten Probeneinführungsposition zum Gleiten entlang eines führenden gegenüberliegenden Paares von Objektträger-Hubspuren (28) in der Gehäuseumfangsfläche (17) hat, die jeweils mindestens einen geneigten Hubschenkel besitzen, und einen nachlaufenden manuellen Objektträger-Anhebe-Aktuator unterhalb des Objektträgers in seiner anfänglichen untersten Probeneinführungsposition zum Gleiten entlang eines nachlaufenden gegenüberliegenden Paars von Objektträger-Hubspuren in der Gehäuseumfangsfläche, die jeweils mindestens einen geneigten Hubschenkel (47) aufweisen, wobei der führende manuelle Objektträger-Anhebe-Aktuator (44) und der nachlaufende manuelle Objektträger-Anhebe-Aktuator mit Abstand voneinander angebracht sind und entlang ihrer entsprechenden gegenüberliegenden Paare von Objektträger-Hubspuren (28) verschoben werden können, von einer anfänglichen Startposition zu einer endgültigen Untersuchungsposition, wobei die anfängliche Startposition der anfänglichen untersten Probeneinführungsposition entspricht und die endgültige Untersuchungsposition der endgültigen obersten Probenuntersuchungsposition entspricht.

8. Untersuchungsvorrichtung (10A, 10B, 10C, 10D) nach einem der Ansprüche 1 bis 5, wobei das Gehäuse (12) besitzt: i) einen Schlitz (62) in der Gehäuseumfangsfläche (17) unterhalb des Objektträgers (21) in seiner anfänglichen untersten Probeneinführungsposition, um durch diesen Schlitz ein Objektträger-Anhebe-Element-(203) gleitend in das Gehäuse (12) einzuführen; und ii) eine Objektträger-Führungsspur (63) zur Führung des Objektträgers (21) von seiner anfänglichen untersten Probeneinführungsposition zu seiner endgültigen obersten Probenuntersuchungsposition beim gleitenden Einführen des Objektträger-Anhebe-Elements (203) in das Gehäuse.

9. Untersuchungsgerät (200) zur Verwendung mit der Untersuchungsvorrichtung (10A, 10B, 10C, 10C) nach Anspruch 8 zur Gewinnung eines Bildes der Probe, wobei das Untersuchungsgerät (200) das Objektträger-Anhebe-Element (203) zum Einführen in die Untersuchungsvorrichtung (10A, 10B, 10C, 10D) enthält.

10. Untersuchungsgerät (200) nach Anspruch 9, wobei die Untersuchungsvorrichtung (10A, 10B, 10C, 10D) einen Geruchssensor (70) zur Gewinnung von Geruchsinformationen von der Probe enthält.

## Revendications

1. Dispositif d'analyse (10A, 10B, 10C, 10D) destiné à permettre l'examen par radiations électromagnétiques EMR d'un spécimen d'au moins un spectre EMR prédéterminé, ce dispositif d'analyse comprenant :
a) un boîtier portable (12) doté d'une face inférieure de boîtier (16), d'une face supérieure de boîtier (18) opposée à ladite face inférieure de boîtier (16), et d'une face périphérique de boîtier (17),
ledit boîtier (12) comportant un port d'introduction de spécimen (19) dans ladite face périphérique de boîtier (17) pour permettre l'introduction du spécimen à l'intérieur,
ladite face inférieure de boîtier (16) comportant un panneau de face inférieure de boîtier (22) et ladite face supérieure de boîtier (18) comportant un panneau de face supérieure de boîtier (23) en registre avec ledit panneau de face inférieure de boîtier (22) pour définir une ligne d'examen par le dispositif d'analyse,
ledit panneau de face inférieure de boîtier (22) et ledit panneau de face supérieure de boîtier (23) étant transparents à l'au moins un spectre EMR prédéterminé ;
b) une glissière de spécimen (21) ayant un panneau de glissière de spécimen (24) pour au moins un spécimen à déposer pour chaud dessus,
ledit panneau de glissière de spécimen (24) étant transparent à l'au moins spectre EMR prédéterminé et en registre mutuel avec ledit panneau de face supérieure de boîtier (23) et ledit panneau de face inférieure de boîtier (22) le long de ladite ligne d'examen ; et
c) un système d'élévation de glissière de spécimen destiné à élever ladite glissière de spécimen,
ladite glissière de spécimen (21) étant apte à être élevée par glissement par rapport à ladite face inférieure de boîtier depuis une position d'introduction de spécimen la plus basse initiale jusqu'à une position d'examen de spécimen la plus haute finale à distance correspondante depuis et de manière à être adjacente à ladite face supérieure de boîtier (18) une fois que ledit au moins un spécimen a été déposé sur ledit panneau de glissière de spécimen (24) dans ladite position d'introduction de spécimen la plus basse initiale,
et ledit panneau de glissière de spécimen (24) étant apte à être juxtaposé intimement contre ledit panneau de face supérieure de boîtier (23) dans ladite position d'examen de spécimen la plus haute finale pour comprimer ledit au moins un spécimen entre ledit panneau de glissière de spécimen (24) et ledit panneau de face supérieure de boîtier (23).

2. Dispositif d'analyse (10A, 10B, 10C, 10D) selon la revendication 1, dans lequel ladite glissière de spécimen (21) inclut un détecteur chimique de pH (54) pour fournir une indication de couleur visible concernant la valeur de pH du spécimen.

3. Dispositif d'analyse (10A, 10B, 10C, 10D) selon la revendication 1 ou 2, dans lequel ladite face inférieure de boîtier (16) est doublée avec du matériau hygroscopique pour absorber les résidus de liquide déplacé depuis ladite glissière de spécimen (21) suite à l'élévation de ladite glissière de spécimen (21) jusqu'à ladite position d'examen de spécimen la plus haute finale.

4. Dispositif d'analyse (10A, 10B, 10C, 10D) selon l'une quelconque des revendications 1 à 3, dans lequel ladite face supérieure de boîtier (18) inclut un port d'agent réactif liquide (64,66) et un canal d'agent réactif liquide pour guider l'agent réactif liquide jusque sur ladite glissière de spécimen (21) dans ladite position d'introduction de spécimen la plus basse initiale pour réagir avec l'au moins un spécimen sur ledit panneau de glissière de spécimen (24).

5. Dispositif d'analyse (10A, 10B, 10C, 10D) selon l'une quelconque des revendications 1 à 3, dans lequel ledit boîtier (12) inclut un système de pompe d'agent réactif liquide intégré (56) pour pomper de l'agent réactif liquide sur ladite glissière de spécimen en vue de réagir avec l'au moins un spécimen dans ladite position d'introduction de spécimen la plus basse initiale, ledit système de pompe d'agent réactif du fond liquide intégré incluant un réservoir d'agent réactif contenant de l'agent réactif liquide et ladite face supérieur de boîtier incluant un plongeur dépendant dirigé vers le bas, l'élévation de ladite glissière de spécimen poussant ledit plongeur dans ledit réservoir d'agent réactif pour pomper de l'agent réactif liquide depuis ledit réservoir d'agent réactif jusque sur ledit panneau de spécimen.

6. Dispositif d'analyse (10A, 10B, 10C, 10D) selon l'une quelconque des revendications 1 à 5, dans lequel ledit boîtier (12) inclut au moins un actionneur d'élévation de glissière de spécimen actionné manuellement (44) sous ladite glissière de spécimen (21) dans ladite position d'introduction de spécimen la plus basse initiale, ledit actionneur d'élévation de glissière de spécimen actionné manuellement (44) ayant une position d'installation initiale correspondant à ladite position d'introduction de spécimen la plus basse initiale et une position d'examen finale correspondant à ladite position d'introduction de spécimen la plus haute initiale.

7. Dispositif d'analyse (10A, 10B, 10C, 10D) selon la revendication 6, dans lequel ledit boîtier (12) inclut un actionneur d'élévation de glissière de spécimen actionné manuellement meneur (44) sous ladite glissière de spécimen dans ladite position d'introduction de spécimen la plus basse initiale pour glisser le long d'une paire opposée meneuse de pistes d'élévation de glissière de spécimen (28) dans ladite face périphérique de boîtier (17), chacune incluant au moins un pied d'élévation incliné, et
un actionneur d'élévation de glissière de spécimen actionné manuellement suiveur sous ladite glissière de spécimen dans ladite position d'introduction de spécimen la plus basse initiale pour glisser le long d'une paire opposée suiveuse de pistes d'élévation de glissière de spécimen dans ladite face périphérique de boîtier, chacune incluant au moins un pied d'élévation incliné (47), lesdits actionneur d'élévation de glissière de spécimen actionné manuellement meneur (44) et actionneur d'élévation de glissière de spécimen actionné manuellement suiveur étant espacés pour un déplacement manuel le long de leurs paires opposées de pistes d'élévation de glissière de spécimen (28) l'un vers l'autre depuis une position d'installation initiale jusqu'à une position d'examen finale, ladite position d'installation initiale correspondant à ladite position d'introduction de spécimen la plus basse initiale et ladite position d'examen finale correspondant à ladite position d'introduction de spécimen la plus haute initiale

8. Dispositif d'analyse (10A, 10B, 10C, 10D) selon l'une quelconque des revendications 1 à 5, dans lequel ledit boîtier (12) inclut i) une fente (62) dans ladite face périphérique de boîtier (17) sous ladite glissière de spécimen (21) dans ladite position d'introduction de spécimen la plus basse initiale pour une insertion par glissement d'un élément d'élévation de glissière de spécimen (203) dans ledit boîtier (12) et ii) une piste de guidage de glissière de spécimen (63) pour guider ladite glissière de spécimen (21) de ladite position d'introduction de spécimen la plus basse initiale à ladite position d'introduction de spécimen la plus haute initiale pendant ladite insertion par glissement dudit élément d'élévation de glissière de spécimen (203) dans ledit boîtier (12).

9. Appareil d'analyse (200) destiné à être utilisé avec le dispositif d'analyse (10A, 10B, 10C, 10D) selon la revendication 8 pour obtenir une image du spécimen, l'appareil d'analyse (200) incluant ledit élément d'élévation de glissière de spécimen (203) destiné à être inséré dans le dispositif d'analyse (10A, 10B, 10C, 10D).

10. Appareil d'analyse (200) selon la revendication 9, dans lequel le dispositif d'analyse (10A, 10B, 10C, 10D) inclut un capteur d'odeurs (70) destiné à obtenir des informations d'odeur en partant du spécimen.
